(19) Europäisches Patentamt
European Patent Office
Office européen des brevets

(11) Numéro de publication: **0 086 126**
**B1**

(12) # FASCICULE DE BREVET EUROPEEN

(45) Date de publication du fascicule du brevet: **24.07.85**

(51) Int. Cl.⁴: **C 07 D 233/50**, A 61 K 31/415

(21) Numéro de dépôt: **83400128.1**

(22) Date de dépôt: **19.01.83**

(54) **Dérivés d'imidazolidine, leur préparation et leur application en thérapeutique.**

(30) Priorité: **05.02.82 FR 8201877**

(43) Date de publication de la demande: **17.08.83 Bulletin 83/33**

(45) Mention de la délivrance du brevet: **24.07.85 Bulletin 85/30**

(84) Etats contractants désignés: **AT BE CH DE FR GB IT LI LU NL SE**

(56) Documents cités:
**EP - A - 0 017 484**
**FR - A - 2 417 502**

(73) Titulaire: **SYNTHELABO, 58, rue de la Glacière, F-75621 Paris Cedex 13 (FR)**

(72) Inventeur: **Purcell, Thomas, Résidence G. Sand 146 bis Rue d'Aulnay, F-92290 Châtenay-Malabry (FR)**

(74) Mandataire: **Ludwig, Jacques et al, SYNTHELABO Service Brevets 58, rue de la Glacière, F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 086 126 B1

## Description

La présente invention concerne des dérivés d'imidazolidine, leur préparation et leur application en thérapeutique.

Les composés de l'invention répondent à la formule I

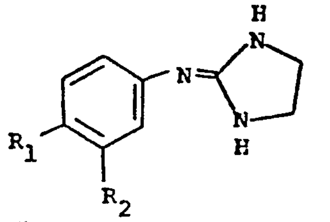

(I)

dans laquelle:

$R_1$ est OH et $R_2$ représente un radical $NHSO_2CH_3$, NHCOR' ou NHCONR'R", R' et R" représentant indépendamment l'un de l'autre un atome d'hydrogène ou un alkyle de 1 à 4 atomes de carbone, ou bien

$R_2$ est OH et $R_1$ représente un radical $NHSO_2CH_3$, NHCOR' ou NHCONR'R", R' et R" représentant indépendamment l'un de l'autre un atome d'hydrogène ou un alkyle de 1 à 4 atomes de carbone.

Les sels pharmacologiquement acceptables des composés I font partie de l'invention.

Les formes tautomères des composés de l'invention font partie de l'invention.

Les composés préférés de l'invention sont ceux dans lesquels $R_1$ est OH et $R_2$ est NHCHO, $NHSO_2CH_3$ ou $NHCONH_2$.

Selon l'invention, on peut préparer les composés I selon les schémas réactionnels suivants, selon les significations de $R_1$ et $R_2$.

*Schéma 1 $R_1$ = OH*

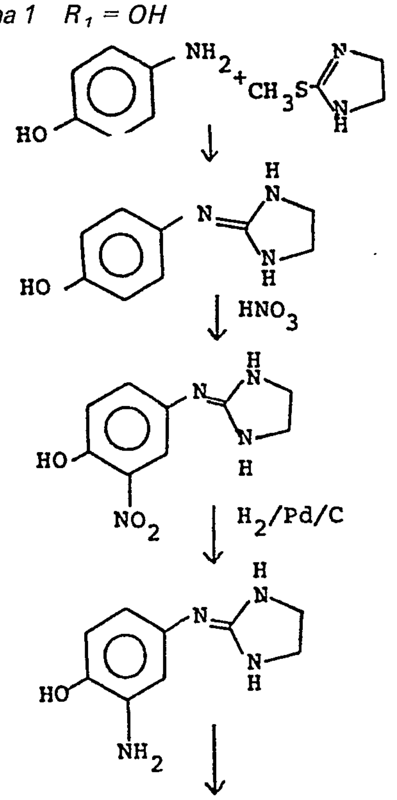


Composé (I)

*Schéma 2 $R_2$ = OH*

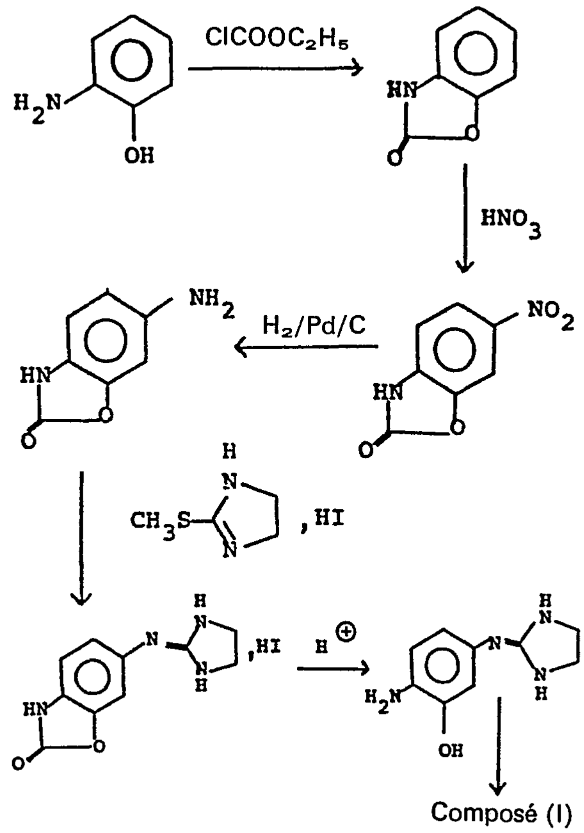


Composé (I)

Selon le schéma réactionnel 1, on fait réagir la p-hydroxyaniline avec la méthylthio-2 imidazoline, par exemple dans de la pyridine pour obtenir l'(hydroxy-4 phénylimino)-2 imidazolidine que l'on nitre à l'aide d'acide nitrique pour obtenir l'(hydroxy-4 nitro-3 phénylimino)-2 imidazolidine que l'on hydrogène, en présence d'un catalyseur d'hydrogénation tel que le charbon palladié afin d'obtenir l'(amino-3 hydroxy-4 phénylimino)-2 imidazolidine que l'on fait réagir soit avec de l'acide formique en présence d'anhydride acétique, soit avec du chlorure de méthylsulfonyle, soit avec le composé NaNCO ou R'NCO (R' = $C_{1-4}$ alkyle).

Selon le schéma réactionnel 2 on fait réagir l'ortho-aminophénol avec le chloroformiate d'éthyle puis on nitre la 3H-benzoxazolone-2 obtenue, on réduit le groupe nitro à l'aide d'hydrogène en présence d'un catalyseur, on fait réagir l'amino-6 3H-benzoxazolone-2 avec la méthylthio-2 imidazoline et on acidifie le composé obtenu pour obtenir l'(amino-4 hydroxy-3 phénylimino)-2 imidazolidine que l'on fait réagir soit avec de l'acide formique en présence d'anhydride acétique, soit avec le chlorure de méthylsulfonyle, soit avec le composé NaNCO ou R'NCO (R' = $C_{1-4}$ alkyle).

Les exemples suivants illustrent l'invention. Les analyses et les spectres IR et RMN confirment la structure des composés.

*Exemple 1:*

*(Formylamino-3 hydroxy-4 phénylimino)-2 imidazolidine et son chlorhydrate*

($R_1$ = OH, $R_2$ = NHCHO)

*1. Iodhydrate d'(hydroxy-4 phénylimino)-2 imidazolidine.*

On porte à la température du reflux pendant 2 h un mélange de 16,35 g (0,15 mol) d'hydroxy-4 phénylamine et de 38,4 g d'iodhydrate de méthylthio-2 imidazoline dans 150 $cm^3$ de pyridine.

On piège le thiol formé par $KMnO_4$ placé dans de l'acide sulfurique dilué. Après repos pendant une nuit on verse le mélange réactionnel sur de l'éther et on le lave avec de l'éther. On reprend le mélange par de l'éthanol. On ajoute de l'éther de pétrole et on essore les cristaux formés.

Le chlorhydrate d'(hydroxy-4 phénylimino)-2 imidazolidine fond à 220-222° C.

*2. Chlorhydrate d'(hydroxy-4 nitro-3 phénylimino)-2 imidazoline.*

On introduit 13,25 g (0,0489 mol) du chlorhydrate d'(hydroxy-4 phénylimino)-2 imidazolidine dans 200 $cm^3$ d'acide acétique à la température ambiante et on ajoute 3,14 $cm^3$ d'acide nitrique (d = 1,42). Au bout de 10 à 15 min on verse le mélange réactionnel dans de l'éther. Après filtration et recristallisation dans de l'éthanol, le chlorhydrate d'(hydroxy-4 nitro-3 phénylimino)-2 imidazolidine fond à 231-233° C.

*3. Chlorhydrate d'(amino-3 hydroxy-4 phénylimino)-2 imidazolidine.*

On hydrogène, dans un appareil de Parr, sous une pression de 34,37 × $10^4$ Pa (50 psi), 3,5 g de chlorhydrate d'(hydroxy-4 nitro-3 phénylimino)-2 imidazolidine dans 80 $cm^3$ de diméthylformamide en présence de charbon palladié à 10%.

Après élimination du catalyseur, on verse le mélange réactionnel dans de l'éther et on décante le surnageant; après addition d'éthanol et d'éther de pétrole au résidu, on essore les cristaux formés.

Le chorhydrate d'(amino-3 hydroxy-4 phénylimino)-2 imidazolidine fond à 217° C.

*4. Chlorhydrate de (formylamino-3 hydroxy-4 phénylimino)-2 imidazolidine.*

Dans un ballon on introduit 5 $cm^3$ d'acide formique et 0,4 $cm^3$ d'anhydride acétique et on agite 15 min à 0° C; puis on ajoute 914 mg (4 mmol) de chlorhydrate d'(amino-3 hydroxy-4 phénylimino)-2 imidazolidine. On agite 30 min et verse le mélange réactionnel dans de l'éther. On essore le précipité et le fait recristalliser dans un mélange éthanol/éther de pétrole.

Le chlorhydrate de (formylamino-3 hydroxy-4 phénylimino)-2 imidazolidine fond à 226° C (déc.).

*Exemple 2:*

*(Aminocarbonylamino-3 hydroxy-4 phénylimino)-2 imidazolidine et son chlorhydrate*

$$(R_1 = OH, R_2 = NHCONH_2)$$

On fait réagir 914 mg (4 mmol) de chlorhydrate d'(amino-3 hydroxy-4 phénylimino)-2 imidazolidine, 260 mg (4,32 mmol) de NaNCO et un mélange de 6 $cm^3$ d'acide acétique et de 4 $cm^3$ d'eau.

On maintient le mélange réactionnel 1 h à 40° C. On évapore à siccité puis reprend par de l'eau. On chromatographie le mélange réactionnel sur une colonne Amberlite IRC50; on reprend le résidu après évaporation par de l'éthanol et ajoute un mélange éther/acide chlorhydrique.

Après recristallisation dans de l'éthanol, le chlorhydrate d'(aminocarbonylamino-3 hydroxy-4 phénylimino)-2 imidazolidine fond à 227° C (déc.)

*Exemple 3:*

*(Formylamino-4 hydroxy-3 phénylimino)-2 imidazolidine et son chlorhydrate*

$$(R_1 = NHCHO, R_2 = OH)$$

*1. 3H-benzoxazolone-2*

A une température comprise entre 0 et 10° C, on ajoute 38,4 $cm^3$ (0,04 mol) de chloroformiate d'éthyle à une solution de 38,4 g (0,35 mol) d'orthominophénol dans 150 $cm^3$ de pyridine. A la fin de l'addition, on agite encore 2 h, puis on verse le mélange réactionnel sur 1,5 l d'éther. Le précipité est essoré. L'éther du filtrat est évaporé au rotovapor. On chauffe le résidu pour distiller la pyridine, jusqu'à 180° C. Après refroidissement, le résidu est recristallisé dans de l'acétate d'éthyle.

F = 136° C

*2. Nitro-6 3H-benzoxazolone-2*

On introduit 35 g (0,26 mol) du composé obtenu sous 1 dans 300 $cm^3$ d'acide acétique et on ajoute tout en agitant, à la température ambiante, 19 $cm^3$ d'acide nitrique (d = 1,42 - 70%). On chauffe progressivement le mélange réactionnel à 100° C et on le maintient 1h à cette température. On refroidit à 20° C et on essore le solide qui est lavé par l'acide acétique puis par de l'éther. Le composé fond à 243° C.

*3. Amino-6 3H-benzoxazolone-2*

On introduit dans l'appareil de Parr sous une pression d'hydrogène de 20,68 × $10^4$ Pa (30 ψ), 10 g du composé obtenu sous 2 en suspension dans 250 $cm^3$ d'éthanol et 1 g de charbon palladié à 10%. On filtre le catalyseur et évapore à sec le filtrat au rotovapor. On lave le résidu avec de l'éthanol. On obtient l'amine qui est utilisée ensuite dans l'étape suivante.

*4. Iodhydrate d'(imidazolidinylidène-2 amino)-6 3H-benzoxazolone-2.*

On porte à la température du reflux dans 100 $cm^3$ de pyridine 13 g (86,7 mmol) du composé obtenu sous 3 et 22,2 g d'iodhydrate de méthylthio-2 imidazoline-2. On poursuit le reflux jusqu'à cessation du dégagement de méthanethiol (2 à 3 h environ). A la fin de la réaction, le mélange refroidi est versé sur 1,5 l d'éther. On lave le résidu gommeux 3 fois avec 300 ml d'éther et on le reprend par 100 $cm^3$ d'éthanol. On essore le produit que l'on fait recristalliser dans du méthanol.

F = 270-272° C

*5. Dichlorhydrate d'(amino-4 hydroxy-3 phénylimino)-2 imidazolidine.*

On dissout 1,2 g du composé obtenu sous 4 dans le minimum d'eau (120 $cm^3$) et on traite par de la résine Amberlite ($Cl^-$) dans du chloroforme. La phase aqueuse est évaporée à sec au rotovapor. Le résidu est repris dans 50 $cm^3$ d'acide chlorhydrique concentré et porté à la température du reflux pendant 32 h environ. Le milieu réactionnel est

évaporé à sec au rotovapor et le résidu est recristallisé dans de l'éthanol.

F = 283° C

*6. Chlorhydrate de (formylamino-4 hydroxy-3 phénylimino)-2 imidazolidine.*

On dissout 2,2 g du composé obtenu sous 5 dans environ 70 $cm^3$ d'eau; on agite la solution obtenue avec une solution de 50 ml de résine Amberlite® L A 2 dans 150 $cm^3$ de toluène. On décante la phase aqueuse, la lave au toluène et l'évapore à sec au rotovapor.

On sèche le résidu sous vide, sur $P_2O_5$. On obtient le monochlorhydrate d'(amino-4 hydroxy-3 phénylimino)-2 imidazolidine.

Dans un ballon de 100 $cm^3$ on introduit 11 $cm^3$ d'acide formique et on ajoute à 0° C, 0,875 $cm^3$ d'anhydride acétique. On agite le mélange réactionnel pendant 15 min à 0° C et on ajoute en une seule fois 2 g du monochlorhydrate obtenu ci-dessus. On agite le mélange réactionnel 30 min à la température ambiante puis on le verse sur de l'éther. On élimine l'éther et lave le résidu 3 fois à l'éther. On dissout le résidu dans de l'éthanol, le traite au noir et le fait cristalliser dans un mélange éthanol/éther de pétrole. On fait recristalliser le produit dans de l'éthanol.

F = 217°C (déc.)

Les composés de l'invention préparés à titre d'exemples sont représentés dans le tableau suivant.

*Tableau*

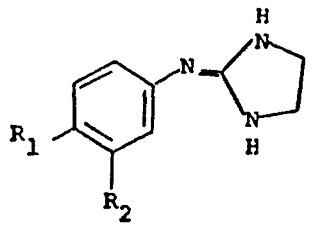

(I)

| Composé | $R_1$ | $R_2$ | F (°C) |
|---|---|---|---|
| 1 | OH | NHCHO | HCl - 226 (déc). |
| 2 | OH | $NHSO_2CH_3$ | HCl - 256 (déc). |
| 3 | OH | $NHCONH_2$ | HCl - 227 (déc). |
| 4 | OH | $NHCONH(CH_2)_3CH_3$ | HCl - 193 |
| 5 | NHCHO | OH | HCl - 217 (déc). |
| 6 | $NHSO_2CH_3$ | OH | HCl - 230-231 |

Les composés de l'invention ont été étudiés pharmacologiquement; leur action sur la sécrétion gastrique a été plus particulièrement étudiée.

La toxicité aiguë des composés de l'invention a été déterminée par voie intrapéritonéale chez des souris mâles de souche CD; la toxicité est supérieure à 100 mg/kg.

L'action sur l'activité gastrique a été déterminée par le test effectué selon la variante de la technique de Shay (Gastroenterology 1945, *5*, 43) décrite par Pascaud et Laubie («Arzeneim. Forsch», 1971, *10*, 1547).

Des rats CD mâles, pesant 200 à 250 g, sont mis à jeun de nourriture solide 48 h avant l'expérience et répartis en blocs randomisés.

Les animaux reçoivent par voir orale 4 ml de sérum physiologique tiède. Ils sont ensuite immédiatement anesthésiés à l'éther. Après laparotomie, le contenu gastrique est évacué par voie duodénale, en pressant légèrement l'estomac, et le pylore est ligaturé. Aussitôt après, le composé à étudier, mis en solution dans du sérum physiologique, est injecté par voie sous-cutanée.

4 h après la ligature du pylore, les animaux sont sacrifiés, l'œsophage est ligaturé et l'estomac est prélevé. Le contenu gastrique est recueilli et centrifugé à 4000 g pendant 3 min. Tout prélèvement contenant du sang ou un résidu solide correspondant à un volume supérieur à 0,6 ml est éliminé. Le volume est mesuré et l'acidité est évaluée à l'aide d'une solution de NaOH titrée de telle sorte que 1 ml correspond à 5 mg d'HCl.

Deux titrages sont effectués:

1. jusqu'au point de virage du diméthylamino-azobenzol (réactif de Töpfer), à pH 3,5 (titrage représentant l'acidité libre);

2. jusqu'au point de virage de la phénolphtaléine à pH 8,5 (titrage représentant l'acidité totale).

Les résultats sont exprimés en µEq/4h/100 g.

Les composés de l'invention diminuent le volume et l'acidité gastrique. A la dose de 0,1 mg/kg la diminution va de 45 à 60%.

Par ailleurs, les composés de l'invention peuvent être actifs dans des applications thérapeutiques où une stimulation des récepteurs α-adrénergiques est exigée; par exemple les applications suivantes sont envisageables:

– vasoconstriction au niveau local, en particulier décongestion nasale,
– hypotension orthostatique d'origine génétique ou médicamenteuse,
– glaucome,
– hypersécrétion gastrique.

L'invention comprend, par conséquent, toutes les compositions pharmaceutiques renfermant les composés de formule générale I et/ou leurs sels pharmacologiquement acceptables comme principes actifs en association avec tout excipient approprié pour l'administration par voie orale ou parentérale.

Les composés I peuvent être présentés sous forme de comprimés, dragées, gélules, capsules, solutions et suspensions buvables, solutés injectables, etc.

La posologie quotidienne peut aller de 0,1 à 10 mg.

**Revendications** pour les Etats contractants BE, CH, DE, FR, GB, IT, LI, LU, NL, SE

1. Dérivés d'imidazolidine répondant à la formule (I)

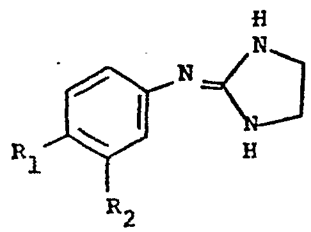

(I)

dans laquelle:

$R_1$ est OH et $R_2$ représente un radical $NHSO_2CH_3$, NHCOR' ou NHCONR'R", R' et R" représentant indépendamment l'un de l'autre, un atome d'hydrogène ou un alkyle de 1 à 4 atomes de carbone, ou bien

$R_2$ est OH et $R_1$ représente un radical $NHSO_2CH_3$, NHCOR' ou NHCONR'R", R' et R" représentant indépendamment l'un de l'autre, un atome d'hydrogène ou un alkyle de 1 à 4 atomes de carbone,

ainsi que leurs sels pharmacologiquement acceptables.

2. Dérivés selon la revendication 1, dans lesquels $R_1$ est OH et $R_2$ a les significations données ci-dessus.

3. Dérivés selon la revendication 2, dans lesquels $R_1$ est OH et $R_2$ est le radical NHCHO, $NHSO_2CH_3$ ou $NHCONH_2$.

4. La (formylamino-3 hydroxy-4 phénylimino)-2 imidazolidine et ses sels pharmacologiquement acceptables.

5. Procédé de préparation des composés selon la revendication 1, dans lesquels $R_1$ est OH, procédé caractérisé en ce que l'on fait réagir la p-hydroxyaniline avec la méthylthio-2 imidazoline, par exemple dans de la pyridine pour obtenir l'(hydroxy-4 phénylimino)-2 imidazolidine que l'on nitre à l'aide d'acide nitrique pour obtenir l'(hydroxy-4 nitro-3 phénylimino)-2 imidazolidine que l'on hydrogène, en présence d'un catalyseur d'hydrogénation tel que le charbon palladié afin d'obtenir l'(amino-3 hydroxy-4 phénylimino)-2 imidazolidine que l'on fait réagir soit avec de l'acide formique en présence d'anhydride acétique, soit avec du chlorure de méthylsulfonyle soit avec le composé NaNCO ou R' NCO (R' = $C_{1-4}$ alkyle).

6. Procédé de préparation des composés selon la revendication 1, dans lesquels $R_2$ est OH, procédé caractérisé en ce que l'on fait réagir l'orthoaminophénol avec le chloroformiate d'éthyle, puis on nitre la 3H-benzoxazoline-2 obtenue, on réduit le groupe nitro à l'aide d'hydrogène en présence d'un catalyseur, on fait réagir l'amino-6 3H-benzoxazolone-2 avec la méthylthio-2 imidazoline et on acidifie le composé obtenu pour obtenir l'(amino-4 hydroxy-3 phénylimino)-2 imidazolidine que l'on fait réagir soit avec de l'acide formique en présence d'anhydride acétique, soit avec le chlorure de méthylsulfonyle, soit avec le composé NaNCO ou R'NCO (R' = $C_{1-4}$ alkyle).

7. Médicament, caractérisé en ce qu'il contient un composé selon l'une des revendications 1 à 4.

8. Composition pharmaceutique, caractérisée en ce qu'elle contient un composé selon l'une des revendications 1 à 4 en association avec tout excipient approprié.

**Revendication** pour l'Etat contractant: AT

Procédé de préparation de dérivés d'imidazolidine répondant à la formule (I)

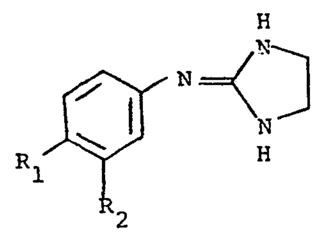

(I)

dans laquelle:

$R_1$ est OH et $R_2$ représente un radical $NHSO_2CH_3$, NHCOR' ou NHCONR'R", R' et R" représentant indépendamment l'un de l'autre, un atome d'hydrogène ou un alkyle de 1 à 4 atomes de carbone, ou bien

$R_2$ est OH et $R_1$ représente un radical $NHSO_2CH_3$, NHCOR' ou NHCONR'R", R' et R" représentant indépendamment l'un de l'autre, un atome d'hydrogène ou un alkyle de 1 à 4 atomes de carbone, procédé caractérisé en ce que, lorsque $R_1$ est OH, on fait réagir la p-hydroxy-aniline avec la méthylthio-2 imidazoline, par exemple dans de la pyridine pour obtenir l'(hydroxy-4 phénylimino)-2 imidazolidine que l'on nitre à l'aide d'acide nitrique pour obtenir l'(hydroxy-4 nitro-3 phénylimino)-2 imidazolidine que l'on hydrogène, en présence d'un catalyseur d'hydrogénation tel que le charbon palladié afin d'obtenir l'(amino-3 hydroxy-4 phénylimino)-2 imidazolidine que l'on fait réagir soit avec de l'acide formique en présence d'anhydride acétique, soit avec du chlorure de méthylsulfonyle, soit avec le composé NaNCO ou R'NCO (R' = $C_{1-4}$ alkyle), ou bien, lorsque $R_2$ est OH, on fait réagir l'orthoaminophénol avec le chloroformiate d'éthyle, puis on nitre la 3H-benzoxazoline-2 obtenue, on réduit le groupe nitro à l'aide d'hydrogène en présence d'un catalyseur, on fait réagir l'amino-6 3H-benzoxazolone-2 avec la méthylthio-2 imidazoline et on acidifie le composé obtenu pour obtenir l'(amino-4 hydroxy-3 phénylimino)-2 imidazolidine que l'on fait réagir soit avec de l'acide formique en présence d'anhydride acétique, soit avec le chlorure de métylsulfonyle,

soit avec le composé NaNCO ou R'NCO (R' = $C_{1-4}$ alkyle).

**Patentansprüche** für die Vertragsstaaten BE, CH, DE, FR, GB, IT, LI, LU, NL, SE

1. Imidazolidin-Derivate der Formel (I)

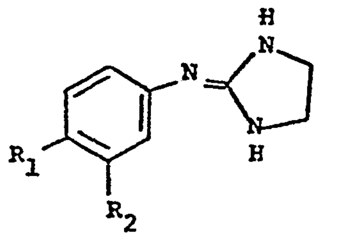

(I)

in welcher:

$R_1$ für OH und $R_2$ für einen Rest $NHSO_2CH_3$, NHCOR' oder NHCONR'R" steht, wobei R' und R" unabhängig voneinander ein Wasserstoffatom oder einen Alkylrest mit 1 bis 4 Kohlenstoffatomen darstellen; oder aber $R_2$ für OH und $R_1$ für einen Rest $NHSO_2CH_3$, NHCOR' oder NHCOR'R" steht, wobei R' und R" unabhängig voneinander ein Wasserstoffatom oder einen Alkylrest mit 1 bis 4 Kohlenstoffatomen darstellen, sowie ihre pharmakologisch anwendbaren Salze.

2. Derivate nach Anspruch 1, in welchen $R_1$ für OH steht und $R_2$ die oben genannten Bedeutung hat.

3. Derivate nach Anspruch 2, in welchen $R_1$ für OH steht und $R_2$ den Rest NHCHO, $NHSO_2CH_3$ oder $NHCONH_2$ bedeutet.

4. 2-(3-Formylamino-4-hydroxyphenylimino)-imidazolidin und seine pharmakologisch anwendbaren Salze.

5. Verfahren zur Herstellung von Verbindungen nach Anspruch 1, bei welcher $R_1$ für OH steht, dadurch gekennzeichnet, dass man p-Hydroxyanilin mit 2-Methylthioimidazolin, beispielsweise in Pyridin, zur Gewinnung von 2-(4-Hydroxyphenylimino)imidazolidin umsetzt, welches man mit Hilfe von Salpetersäure zur Herstellung von 2-(4-Hydroxy-3-nitrophenylimino)imidazolidin nitriert, welches man in Gegenwart eines Hydrierkatalysators, wie Palladium auf Aktivkohle, zur Gewinnung von 2-(3-Amino-4-hydroxyphenylimino)imidazolidin hydriert, welches man seinerseits entweder mit Ameisensäure in Gegenwart von Essigsäureanhydrid oder mit Methylsulfonylchlorid oder mit der Verbindung NaNCO oder R'NCO (R' = $C_{1-4}$-Alkyl) umsetzt.

6. Verfahren zur Herstellung von Verbindungen nach Anspruch 1, in welchen $R_2$ für OH steht, dadurch gekennzeichnet, dass man o-Aminophenol mit Äthylchloroformiat umsetzt, dass das erhaltene 2-3H-Benzoxazolin nitriert, die Nitrogruppe mit Hilfe von Wasserstoff in Gegenwart eines Katalysators reduziert, das 6-Amino-2-3H-Benzoxazolidin mit 2-Methylthioimidazolin umsetzt und die erhaltene Verbindung ansäuert, um das 2-(4-Amino-3-hydroxyphenylimino)imidazolidin zu erhalten, welches man entweder mit Ameisensäure in Gegenwart von Essigsäureanhydrid oder mit Methylensulfonylchlorid oder mit der Verbindung NaNCO oder R'NCO (R' = $C_{1-4}$-Alkyl) umsetzt.

7. Arzneimittel, dadurch gekennzeichnet, dass es eine Verbindung nach irgendeinem der Ansprüche 1 bis 4 enthält.

8. Pharmazeutische Zusammensetzung, dadurch gekennzeichnet, dass sie eine Verbindung nach irgendeinem der Ansprüche 1 bis 4 in Kombination mit einem geeigneten Träger enthält.

**Patentanspruch** für den Vertragsstaat: AT

Verfahren zur Herstellung von Imidazolidin-Derivaten der Formel (I)

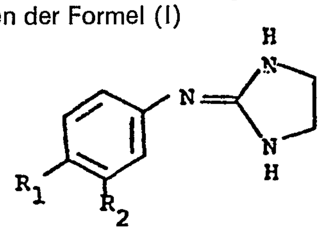

(I)

in welcher:

$R_1$ für OH steht und $R_2$ einen Rest $NHSO_2CH_3$, NHCOR' oder NHCONR'R" bedeutet, wobei R' und R" unabhängig voneinander ein Wasserstoffatom oder ein Alkyl mit 1 bis 4 Kohlenstoffatomen darstellen; oder aber $R_2$ für OH steht und $R_1$ einen Rest $NHSO_2CH_3$, NHCOR' oder NHCONR" bedeutet, wobei R' und R" unabhängig voneinander ein Wasserstoffatom oder ein Alkyl mit 1 bis 4 Kohlenstoffatomen darstellen, welches Verfahren dadurch gekennzeichnet ist, dass man bei $R_1$ gleich OH p-Hydroxyanilin mit 2-Methylthioimidazolin, beispielsweise in Pyridin, reagieren lässt, um 2-(4-Hydroxyphenylimino)imidazolidin zu erhalten, das man mit Hilfe von Salpetersäure zu 2-(4-Hydroxy-3-nitrophenylimino)imidazolidin nitriert, das man in Gegenwart eines Hydrierkatalysators, wie Palladium-Aktivkohle, zur Herstellung von 2-(3-Amino-4-hydroxyphenylimino)imidazolidin hydriert, welches man seinerseits entweder mit Ameisensäure in Gegenwart von Essigsäureanhydrid oder mit Methylsulfonychlorid oder mit der Verbindung NaNCO oder R'NCO (R' = $C_{1-4}$-Alkyl) zur Reaktion bringt, oder aber, bei $R_2$ gleich OH, o-Aminophenol mit Äthylchloroform reagieren lässt, dann das erhaltene 2-3H-Benzoxazolon nitriert, die Nitrogruppe mit Hilfe von Wasserstoff in Gegenwart eines Katalysator reduziert, das 6-Amino-2-3H-benzoxazolon mit 2-Methylthioimidazolin reagieren lässt und die erhaltene Verbindung ansäuert, um 2-(4-Amino-3-hydroxyphenylimino)imidazolidin zu erhalten, das man entweder mit Ameisensäure in Gegenwart von Essigsäureanhydrid oder mit Methylsulfonylchlorid oder mit der Verbindung NaNCO oder R'NCO (R' = $C_{1-4}$-Alkyl) umsetzt.

**Claims** for the contracting States BE, CH, DE, FR, GB, IT, LI, LU, NL, SE

1. Imidazolidine derivatives responding to Formula (I)

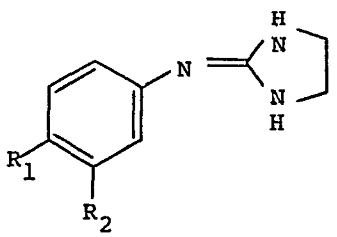

(I)

wherein

$R_1$ is OH and $R_2$ represents a $NHSO_2CH_3$, NHCOR' or NHCONR'R" radical, R' and R" independently of one another each representing a hydrogen atom or an alkyl having 1 to 4 carbon atoms; or

$R_2$ is OH and $R_1$ represents a $NHSO_2CH_3$, NHCOR' or NHCONR'R" radical, R' and R" independently of one another each representing a hydrogen atom or an alkyl having 1 to 4 carbon atoms, and their pharmacologically acceptable salts.

2. Derivatives according to Claim 1, wherein $R_1$ is OH and $R_2$ is as defined above.

3. Derivatives according to Claim 2, wherein $R_1$ is OH and $R_2$ is the NHCHO, $NHSO_2CH_3$ or $NHCONH_2$ radical.

4. 2-(3-Formylamino-4-hydroxy-phenylimino)-imidazolidine and its pharmacologically acceptable salts.

5. A process for the preparation of the compounds according to Claim 1 wherein $R_1$ is OH, characterized in that p-hydroxy-aniline is reacted with 2-methylthio-imidazoline, for exemple in pyridine medium, so as to produce 2-(4-hydroxy-phenylimino)imidazolidine, which is nitrated with nitric acid so as to produce 2-(4-hydroxy-3-nitro-phenylimino)imidazoline, which is hydrogenated in the presence of a hydrogenation catalyst such as palladium charcoal, so as to produce 2-(3-amino-4-hydroxy-phenylimino)imidazolidine, which is reacted either with formic acid in the presence of acetic anhydride, or which methylsulfonyl chloride, or with the compound NaNCO or R'NCO (R' = $C_{1-4}$alkyl).

6. A process for the preparation of the compounds according to Claim 1 wherein $R_2$ is OH, characterized in that ortho-amino-phenol is reacted with ethyl chloroformiate, then the 3H-benzoxazol-2-one obtained is nitrated, the nitro group is reduced with hydrogen in the presence of a catalyst, the 6-amino-3H-benzoxazol-2-one is reacted with 2-methylthio-imidazoline and the compound obtained is acidified so as to produce 2-(4-amino-3-hydroxy-phenylimino)imidazoline which is reacted either with formic acid in the presence of acetic anhydride, or with methylsulfonyl chloride, or with the compound NaNCO or R'NCO (R' = $C_{1-4}$alkyl).

7. A medicament, characterized in that it contains a compound according to any one of Claims 1 to 4.

8. Pharmaceutical composition, characterized in that it contains a compound according to anyone of Claims 1 to 4 in association with any suitable excipient.

**Claim** for the contracting State: AT

A process for the preparation of imidazolidine derivatives responding to Formula (I)

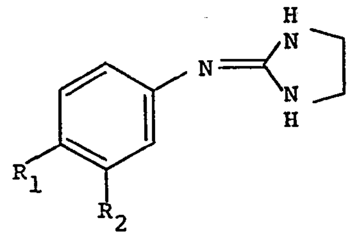

(I)

wherein

$R_1$ is OH and $R_2$ represents a $NHSO_2CH_3$, NHCOR' or NHCONR'R" radical, R' and R" independently of one another each representing a hydrogen atom or an alkyl having 1 to 4 carbon atoms; or

$R_2$ is OH and $R_1$ represents a $NHSO_2CH_3$, NHCOR' or NHCONR'R" radical, R' and R" independently of one another each representing a hydrogen atom or an alkyl having 1 to 4 carbon atoms, process characterized in that, when $R_1$ is OH, p-hydroxyaniline is reacted with 2-methylthio-imidazoline, for exemple in pyridine medium, so as produce 2-(4-hydroxyphenylimino)imidazolidine, which is nitrated with nitric acid so as to produce 2-(4-hydroxy-3-nitro-phenylimino)-imidazoline, which is hydrogenated in the presence of a hydrogenation catalyst such as palladium charcoal, so as to produce 2-(3-amino-4-hydroxy-phenylimino)imidazolidine, which is reacted either with formic acid in the presence of acetic anhydride, or with methylsulfonyl chloride, or with the compound NaNCO or R'NCO (R' = $C_{1-4}$alkyl), or, when $R_2$ is OH, ortho-aminophenol is reacted with ethyl chloroformiate, then the 3H-benzoxazol-2-one obtained is nitrated, the nitro group is reduced with hydrogen in the presence of a catalyst, the 6-amino-3H-benzoxazol-2-one is reacted with 2-methylthio-imidazoline and the compound obtained is acidified so as to produce 2-(4-amino-3-hydroxyphenylimino)imidazoline which is reacted either with formic acid in the presence of acetic anhydride, or with methylsulfonyl chloride, or with the compound NaNCO or R'NCO (R' = $C_{1-4}$alkyl).